(19) Europäisches Patentamt
European Patent Office
Office européen des brevets

(11) **EP 1 642 133 B1**

(12) **EUROPEAN PATENT SPECIFICATION**

(45) Date of publication and mention
of the grant of the patent:
**09.04.2014 Bulletin 2014/15**

(21) Application number: **04740556.8**

(22) Date of filing: **30.06.2004**

(51) Int Cl.:
*G01N 33/574* (2006.01)

(86) International application number:
**PCT/EP2004/007195**

(87) International publication number:
**WO 2005/003776 (13.01.2005 Gazette 2005/02)**

(54) **IN VITRO METHODS FOR DETECTING RENAL CANCER**

IN VITRO VERFAHREN ZUM NACHWEIS VON NIERENKREBS

PROCEDE IN VITRO POUR LA DETECTION DU CANCER DU REIN

(84) Designated Contracting States:
**AT BE BG CH CY CZ DE DK EE ES FI FR GB GR HU IE IT LI LU MC NL PL PT RO SE SI SK TR**

(30) Priority: **30.06.2003 ES 200301518**

(43) Date of publication of application:
**05.04.2006 Bulletin 2006/14**

(73) Proprietor: **Oncomatryx Biopharma, S.L.**
**48160 Derio (Vizcaya) (ES)**

(72) Inventors:
• **GÓMEZ ROMÁN, José Javier**
**2a Planta,**
**48160 Derio (ES)**
• **SÁENZ JIMÉNEZ, Maria Pilar**
**2a Planta,**
**48160 Derio (ES)**
• **OCHOA GARAY, Jorge**
**2a Planta,**
**48160 Derio (ES)**
• **DEL AMO IRIBARREN, Jokin**
**2a Planta,**
**48160 Derio (ES)**
• **SANZ IBAYONDO, Cristina**
**2a Planta,**
**48160 Derio (ES)**
• **JUNQUERA SANCHEZ-VALLEJO, Corina**
**2a Planta,**
**48160 Derio (ES)**
• **SIMÓN BUELA, Laureano**
**2a Planta,**
**48160 Derio (ES)**

• **MARTÍINEZ MARTÍNEZ, Antonio**
**2a Planta,**
**48160 Derio (ES)**
• **ARGÜELLES SÁNCHEZ, Maria Eladia**
**2a Planta,**
**48160 Derio (ES)**
• **VAL BERNAL, José Fernando**
**2a Planta,**
**48160 Derio (ES)**
• **CUEVAS GONZÁLEZ, Jorge**
**2a Planta,**
**48160 Derio (ES)**

(74) Representative: **ABG Patentes, S.L.**
**Avenida de Burgos, 16D**
**Edificio Euromor**
**28036 Madrid (ES)**

(56) References cited:
WO-A-01/14420          WO-A-03/031930
WO-A-2004/050914      WO-A1-97/17368
WO-A1-2003/035100

• **CIRCOSTA PAOLA ET AL: "CD100/Plexin-B1 interaction emphasizes the role of bone marrow microenvironment in B-CLL" BLOOD, vol. 98, no. 11 Part 1, 16 November 2001 (2001-11-16), page 360a XP009041247 & 43RD ANNUAL MEETING OF THE AMERICAN SOCIETY OF HEMATOLOGY, PART 1; ORLANDO, FLORIDA, USA; DECEMBER 07-11, 2001 ISSN: 0006-4971**

• GIESEG MICHAEL A ET AL: "Expression profiling of human renal carcinomas with functional taxonomic analysis." BMC BIOINFORMATICS [ELECTRONIC RESOURCE]. 30 SEP 2002, vol. 3, no. 26, 30 September 2002 (2002-09-30), XP002309629 ISSN: 1471-2105

SEQUENCE LISTING

<110> PROGENIKA BIOPHARMA S.A.

<120> In vitro methods for detecting renal cancer

<160> 23

<170> PatentIn version 3.1

<210> 1
<211> 20
<212> DNA
<213> Artificial sequence

<220> synthetic DNA
<223> direct primer designed to amplify, in combination with SEQ ID
NO : 2, cDNA of the plexin-B1 gene

<400> 1
acagtgtgac aggcaaggcc          20

<210> 2
<211> 23
<212> DNA
<213> Artificial sequence

<220> synthetic DNA
<223> reverse primer designed to amplify, in combination with SEQ ID
NO : 1, cDNA of the plexin-B1 gene

<400> 2
cacagccaat agtgcattca agg          23

<210> 3

<211> 25
<212> DNA
<213> Artificial sequence

<220> synthetic DNA
<223> probe sequence of the 33783_at of Affymetrix, the position of said probe in the mRNA sequence of the plexin-B1 gene being 6508

<400> 3
ttcagcctgg cctgggcagc cctgg        25

<210> 4
<211> 25
<212> DNA
<213> Artificial sequence

<220> synthetic DNA
<223> probe sequence of the 33783_at of Affymetrix, the position of said probe in the mRNA sequence of the plexin-B1 gene being 6545

<400> 4
gaggccacct tcttaggtgc ctgta        25

<210> 5
<211> 25
<212> DNA
<213> Artificial sequence

<220> synthetic DNA
<223> probe sequence of the 33783_at of Affymetrix, the position of said probe in the mRNA sequence of the plexin-B1 gene being 6563

<400> 5
gcctgtagtg actgacaagc agagt        25

<210> 6
<211> 25
<212> DNA
<213> Artificial sequence

<220> synthetic DNA
<223> probe sequence of the 33783_at of Affymetrix, the position of said probe in the mRNA sequence of the plexin-B1 gene being 6565

<400> 6
ctgtagtgac tgacaagcag agtta        25

<210> 7
<211> 25
<212> DNA
<213> Artificial sequence

<220> synthetic DNA
<223> probe sequence of the 33783_at of Affymetrix, the position of said probe in the mRNA sequence of the plexin-B1 gene being 6651

<400> 7
agacccgggg cctcaaggct catgg        25

<210> 8
<211> 25
<212> DNA
<213> Artificial sequence

<220> synthetic DNA
<223> probe sequence of the 33783_at of Affymetrix, the position of said probe in the mRNA sequence of the plexin-B1 gene being 6659

<400> 8
ggcctcaagg ctcatggggt agtac        25

<210> 9
<211> 25
<212> DNA
<213> Artificial sequence

<220> synthetic DNA
<223> probe sequence of the 33783_at of Affymetrix, the position of said probe in the mRNA sequence of the plexin-B1 gene being 6670

<400> 9
tcatggggta gtacccagcc tgctc        25

<210> 10
<211> 25
<212> DNA
<213> Artificial sequence

<220> synthetic DNA
<223> probe sequence of the 33783_at of Affymetrix, the position of said probe in the mRNA sequence of the plexin-B1 gene being 6704

<400> 10
agcgaccctg tgacaccggt ctgca        25

<210> 11
<211> 25
<212> DNA
<213> Artificial sequence

<220> synthetic DNA
<223> probe sequence of the 33783_at of Affymetrix, the position of said probe in the mRNA sequence of the plexin-B1 gene being 6706

<400> 11
cgaccctgtg acaccggtct gcagg        25

<210> 12
<211> 25
<212> DNA
<213> Artificial sequence

<220> synthetic DNA
<223> probe sequence of the 33783_at of Affymetrix, the position of said probe in the mRNA sequence of the plexin-B1 gene being 6809

<400> 12

ctggccttgg ccacactggg attcg        25

<210> 13
<211> 25
<212> DNA
<213> Artificial sequence

<220> synthetic DNA
<223> probe sequence of the 33783_at of Affymetrix, the position of said probe in the mRNA sequence of the plexin-B1 gene being 6812

<400> 13
gccttggcca cactgggatt cggag        25

<210> 14
<211> 25
<212> DNA
<213> Artificial sequence

<220> synthetic DNA
<223> probe sequence of the 33783_at of Affymetrix, the position of said probe in the mRNA sequence of the plexin-B1 gene being 6843

<400> 14
gaggagagcc ccatgcttcc tgtct        25

<210> 15
<211> 25
<212> DNA
<213> Artificial sequence

<220> synthetic DNA
<223> probe sequence of the 33783_at of Affymetrix, the position of said probe in the mRNA sequence of the plexin-B1 gene being 6845

<400> 15
ggagagcccc atgcttcctg tctgc        25

<210> 16
<211> 25
<212> DNA
<213> Artificial sequence

<220> synthetic DNA
<223> probe sequence of the 33783_at of Affymetrix, the position of said probe in the mRNA sequence of the plexin-B1 gene being 6997

<400> 16
acagggctgc cctgcctcat aggta        25

<210> 17
<211> 25
<212> DNA
<213> Artificial sequence

<220> synthetic DNA
<223> probe sequence of the 33783_at of Affymetrix, the position of said probe in the mRNA sequence of the plexin-B1 gene being 7009

<400> 17
tgcctcatag gtagccatgg tgagg        25


<210> 18
<211> 25
<212> DNA
<213> Artificial sequence


<220> synthetic DNA
<223> probe sequence of the 33783_at of Affymetrix, the position of
said probe in the mRNA sequence of the plexin-B1 gene being 7061


<400> 18
agagtggtga ctccattgac ccagc        25


<210> 19
<211> 21
<212> DNA
<213> Artificial sequence


<220> synthetic DNA
<223> direct primer designed to amplify, in combination with SEQ ID
NO : 20, a fragment of human plexin-B1 located at the 3'end of the coding sequence


<400> 19
tcaacgcgga cagttcaagt a        21


<210> 20
<211> 20
<212> DNA
<213> Artificial sequence


<220> synthetic DNA
<223> reverse primer designed to amplify, in combination with SEQ ID
NO : 19, a fragment of human plexin-B1 located at the 3'end of the coding sequence


<400> 20
cacggacgca tatctcacgt        20


<210> 21
<211> 17
<212> DNA
<213> Artificial sequence


<220> synthetic DNA
<223> direct primer designed to amplify, in combination with SEQ ID
NO : 22, a fragment of rib I10 gene used as a control in the RT-PCR reaction


<400> 21
tgcgatggct gcacaca        17


<210> 22
<211> 23
<212> DNA
<213> Artificial sequence


<220> synthetic DNA
<223> reverse primer designed to amplify, in combination with SEQ ID

NO : 21, a fragment of rib I10 gene used as a control in the RT-PCR reaction

<400> 22
tcccttagag caacccatac aac          23

<210> 23
<211> 15
<212> PRT
<213> Artificial sequence
<223> Peptide containing residues 1113-1127 of human plexin-B1

<400> 23

```
        Cys Ala Val Asp Ala Gln Glu Tyr Glu Val Ser Ser Ser Leu Val
          1               5               10                  15
```

**Description**

**FIELD OF THE INVENTION**

The present invention refers to *an in vitro* method for detecting the presence of renal cancer in an individual, for determining the stage, malignancy or severity of said carcinoma in the individual or for monitoring the effect of the therapy administered to an individual having said cancer; to the search, identification, development and assessment of the efficacy of compounds for therapy for said cancer in an attempt to develop new drugs; as well as to agents inducing Plexin-B1 protein expression and/or activity, or to agents inhibiting the effects of Plexin-B1 protein expression and/or activity repression.

**BACKGROUND OF THE INVENTION**

Despite all the advances which have been made in recent years, cancer is still one of the main causes of death worldwide. More than 5 million new cancer cases are diagnosed worldwide each year, and more than 3.5 million deaths are caused by several types of cancer, according to data provided by the International Agency for Research on Cancer, GLOBOCAN, from the year 2000.

Renal neoplasms are frequent tumors in western societies; they represent 2% of all cancer cases. According to data from the International Agency for Research on Cancer, GLOBOCAN, from the year 2000, more than 90,000 new cases are diagnosed each year in Europe, 9,000 in Japan and 30,000 in North America; more than 39,000, 4,000 and 12,000 deaths are caused each year by renal carcinoma in Europe, Japan and North America, respectively; and worldwide mortality caused by renal carcinoma exceeds 100,000 cases per year.

Advances in knowledge of cancer genetics have allowed for the classification of several types of renal tumors. The most common subtype is the conventional renal cell carcinoma, which is different from the papillary, chromophobe or collecting duct subtypes. Renal oncocytoma is a benign neoplasm at times undistinguishable from renal carcinoma, which is a malignant neoplasm. Prior studies have shown that all these histological subtypes are genetically and biologically different, and that both their morphology and behavior are determined by distinctive molecular factors (Kovacs G., et al., J. Pathol., 1997, 183:131-133).

Renal cancer diagnosis systems are currently based on the identification of clinical symptoms and on radiological techniques. The most common symptoms are hematuria (in 50-60% of patients), abdominal pain (in 40% of patients) and a palpable mass in the flank of the abdomen (in 30-40% of patients) (Ritchie A.W.S. and Chisholm G.D., Semin. Oncol., 1983, 10:390-400). The simultaneous combination of these symptoms ("classic triad") occurs in less than 10% of patients. Small, localized tumors rarely cause symptoms, delaying diagnosis until advanced stages. 25% of the tumors are diagnosed by means of computer tomography and ultrasonography (Porena M., et al., J. Clin. Ultrasound, 1992, 20:395-400; Konnack J.W. and Grossman H.B., J. Urol., 1985, 134:1094-1096; Thompson I.M. and Peek M., J. Urol., 1988, 140:487-490); tumors diagnosed with these techniques are usually smaller than those causing symptoms and are more easily resectable to achieve healing (Konnack J.W. and Grossman H.B., J. Urol., 1985, 134:1094-1096; Thompson I.M. and Peek M., J. Urol., 1988, 140:487-490; Smith S.J., et al., Radiology, 1989, 170:699-703).

Alteration of gene expression levels is closely related to uncontrolled cell growth and to de-differentiation processes, common occurrences in all types of cancer. Expression levels of the so-called "tumor suppressing genes", which act to prevent malignant cell growth, are repressed in the tumor cells; and expression levels of the so-called "oncogenes", which act to induce malignant growth, are higher in tumor cells.

Many genes have been associated to the development of renal cancer, such as the von Hippel-Lindau gene (Seizinger B.R., et al., Nature, 1988, 332:268-269), the epidermal growth factor receptor gene (Ishikawa J., et al., Int. J. Cancer, 1990, 45:1018-1021), the transforming growth factor-alpha gene (Lager D.J., et al., Mod. Pathol., 1994, 7:544), the c-myc oncogene (Yao M., et al., Cancer Res., 1988, 48:6753-6757), retinoid metabolism genes (Guo X., et al., Cancer Res., 2001, 61:2774-2781), or cell adhesion factor genes (Kuroiwa, K., et al., J. Surg. Oncol., 2001, 77:123-131). However, many of the genes involved in the beginning and in the progression of renal tumors are still unknown.

There is currently no non-invasive *in vitro* method detecting renal tumors with acceptable sensitivity and specificity levels; a highly sensitive and specific non-invasive method would allow for routinely carrying out clinical analyses for detecting these tumors. The identification of genes differentially expressed in renal carcinomas could allow for the identification of biological markers, which could be highly valuable for the *in vitro* diagnosis, *in vitro* prognosis and treatment of this disease (Boer J.M., et al., Genome Res., 2001, 11:1861-1870). The detection of changes in the gene expression level or in the concentration of encoded proteins in body fluids could be the basis of non-invasive *in vitro* diagnosis renal cancer methods.

Once the patient has been diagnosed with renal cancer, surgical resection is the treatment of choice. Radical nephrectomy, including resection of the kidney, perirenal fat and the ipsilateral adrenal gland, has generally been carried out since the 1960s (Robson C.J., et al., J. Urol, 1969, 101:297-301). Nephron-sparing surgery has been used to treat small, localized tumors (Herr H.W., Cancer, 1994, 73:160-162). The most important parameter for predicting survival is the anatomical extension of the tumor. Patients with small, localized tumors which are completely resected have higher survival rates than those having affected nodes or distal metastasis. 20-30% of patients with localized tumors recover after radical nephrectomy; less than 5% experience local recidivations and 50-60% of patients develop distant metastasis (Rabinovitch R.A., et al., J. Clin. Oncol., 1994, 12:206-212; Sandock, D.S., et al., J. Urol, 1995, 154:28-31). Once metastasis develops, the long-term survival prognosis significantly worsens; currently, 30% of patients diagnosed are already in stage IV, i.e. they have developed distant metastasis.

Treating renal carcinoma is extremely difficult due to its capability of extending without causing symptoms, due to its inherent resistance to conventional systemic chemotherapy and due to the inability of radiotherapy to reduce post-nephrectomy relapse levels, even in patients with affected nodes or tumors not completely resected (Kjaer M., et al., Int. J. Radiat. Oncol. Biol. Phys., 1987, 13:665-672). Almost 50% of patients, and 90-95% of patients with distant metastasis, die during the five years after diagnosis. To combat this reality, molecular genetics studies are providing knowledge of the pathogenesis of this disease and they can provide new targets against which new therapeutic agents can be developed which are more effective than those currently available.

The family of proteins called Plexins is made up of several proteins with cellular cytoplasmic transmembrane domains; Plexins act as semaphorin protein family receptors, although they have also been found expressed in extraneuronal tissues with other molecular functions. Plexin-B1 acts as a receptor of semaphorin III, which is a protein causing neuronal growth cone collapse and the chemical repulsion of axons (Driessens M.H., Olivo C., Nagata K., Inagaki, M., Collard J.G., FEBS lett., 2002, 529:168-172). As of today it has not been proven that Plexins play a role in the carcinogenesis process; however it has been proven that there is a complex and dynamic pattern of interaction between Plexins and small guanosine triphosphatase rho proteins (Castellani V., et al., Curr. Opin. Neurobiol., 2002, 12:532). These Rho-GTPases have a function in reordering the cytoskeleton and are involved in cell adhesion and migration processes; in fact, activation of the Rac protein, one of the members of this family, has been associated with cell migration induction processes and the development of a tumor invasion phenotype (Keely, et al., 1997. Nature 390, 632-637; Sander, et al., J. Cell Biol., 1998, 143:1385-1398). On the other hand, p21 protein-activated kinase (PAK) is the Rac activating factor and competes with Plexin-B 1 in order to bind to Rac; this interaction is two-way given that the Plexin-B1 - Rac interaction inhibits PAK activation (Vikis H.G., Li W., and Guan K.L. Genes Dev., 2002, 16:836-845; Vikis H.G., Li W., and Guan K.L. Proc. Natl. Acad. Sci. USA, 2002, 99:12085-12090). WO 2004/050914 identifies cancer-associated mutations of the plexin-B1 gene which may be useful in screening and diagnosis of cancer and as drug targets in the development of anticancer therapeutics; it also discloses gene therapy of cancer by administration of nucleic acids capable of expressing the wild type form of plexin-B1 and diagnostic kits comprising a primer pair and/or probes for the amplification of the plexin-B1 nucleic acid; however said document lacks a supported disclosure relating to renal cancer. It has also been disclosed, using DNA chips, that the *plexin-B1* gene was one of the 213 genes having reduced expression in renal cell carcinoma samples when compared with healthy kidney samples (Gieseg M.A., Cody T., Man M.Z., Madore S.J., Rubin M.A., and Kalfjian E.P., BMC Bioinformatics, 2002, 3:26); however, the authors of this work did not prove that the differential gene expression levels of Plexin-B1 were maintained when analyzing the samples by methods which arc more sensitive, specific and reliable than DNA-chips, such as by quantitative RT-PCR for example; nor did they prove that the differential gene expression levels translated into differential protein expression levels.

Consistent with this hypothetical function of Plexin-B1, after laborious research and by using different techniques (DNA - chips and quantitative PCR to measure gene expression levels and Western-blot to measure protein expression levels), the authors of the present invention have discovered that interestingly *plexin-B1* gene expression is reduced in renal carcinomas, the reduction being proportional to malignity and invasiveness. Also, the authors of the present invention have discovered that the concentration of the Plexin-B1 protein is also reduced in renal carcinomas. Finally, and yet more interestingly, the authors of the present invention have discovered that the overexpression of Plexin-B1 causes a decrease in cell proliferation of renal carcinoma cells.

The present invention therefore provides *an in vitro* method for detecting the presence of a carcinoma in an individual, for determining the stage or severity of said carcinoma in the individual or for monitoring the effect of the therapy administered to an individual having said carcinoma, based on the detection and/or quantification of the Plexin-B1 protein, of the *plexin-B1* gene mRNA or the corresponding cDNA in a sample from said individual. The present invention also provides a method for treating renal cancer comprising the administration of Plexin-B 1 protein.

## SUMMARY OF THE INVENTION

A first aspect of the present invention is to provide an *in vitro* method to detect the presence of renal cancer in an individual, to determine the stage or severity of said cancer in the individual or to monitor the effect of the therapy administered to an individual with this cancer.

A second aspect of the present invention is an *in vitro* method to screen for, identify, develop and evaluate the efficacy of compounds to treat renal cancer.

An additional aspect of the invention lies in the use of sequences derived from the *plexin-B1* gene in methods to establish the diagnosis and prognosis *in vitro* of renal cancer, and to screen for, identify, develop and evaluate the efficacy of compounds for the treatment of renal cancer.

A further aspect of the present invention consists in the provision of agents that induce Plexin-B 1 protein expression and/or activity, or in that they inhibit the carcinogenic effects of the repression of plexin-B1 protein expression, for treating renal cancer.

Another aspect of the present invention consists in a pharmaceutical composition comprising one or several therapeutic agents together with a pharmaceutically acceptable excipient for treating kidney cancer. One of these agents is the Plexin-B1 protein.

A final aspect of the present invention consists in a kit for carrying out the present invention.

## DESCRIPTION OF THE DRAWINGS

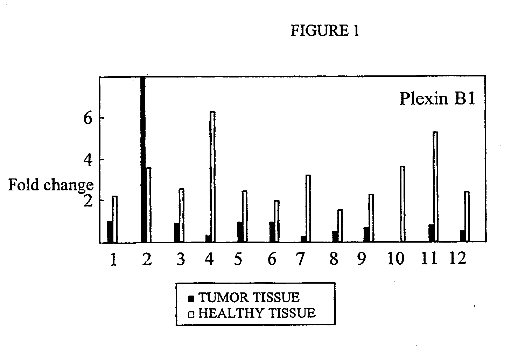

**Figure 1** shows differential plexin-B1 gene expression levels, by real time quantitative RT-PCR, in samples from kidney biopsies of individuals affected with different types of renal carcinomas. The bars represent the plexin-B1 gene expression level compared to the gadph gene (reference gene) expression level in each one of the tumor and non-tumor samples.

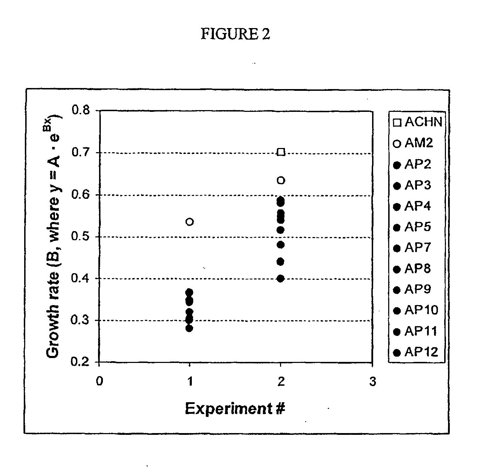

**Figure 2** shows that the growth rate of plexin-B1 expressing clones (AP2-AP12) is slower than the growth of the control clone (AM2) or that of parenteral ACHN cells. The growth rate is represented by B in $y = A \cdot e^{Bx}$, the equation that describes the regression curve of exponentially growing cultures.

## DETAILED DESCRIPTION OF THE INVENTION

To facilitate understanding of the present patent application, the meaning of some terms and expressions in the context of the invention will be explained below:

The terms "subject" or "individual" refer to members of mammalian animal species and includes, but is not limited to, domestic animals, primates and humans; the subject is preferably a human being, male or female, of any age or race.

The term "cancer or carcinoma" refers to the disease which is characterized by an uncontrolled proliferation of abnormal cells capable of invading adjacent tissues and spreading to distant organs.

The term "renal cancer" refers to any proliferative malignant disorder of kidney cells.

The term "tumor" refers to any abnormal tissue mass resulting from a benign (non-cancerous) or malignant (cancerous) neoplastic process.

The term "gene" refers to a molecular chain of deoxyribonucleotides encoding a protein.

The term "DNA" refers to deoxyribonucleic acid. A DNA sequence is a deoxyribonucleotide sequence.

The term "cDNA" refers to a nucleotide sequence complementary of an mRNA sequence.

The term "RNA" refers to ribonucleic acid. An RNA sequence is a ribonucleotide sequence.

The term "mRNA" refers to messenger ribonucleic acid, which is the fraction of total RNA which is translated into proteins.

The phrase "mRNA transcribed from" refers to the transcription of the gene (DNA) into mRNA as a first step for the gene to be expressed and translated to a protein.

The term "sequence of nucleotides" or "nucleotide sequence" indistinctly refers to a ribonucleotide (RNA) or deoxyribonucleotide (DNA) sequence.

The term "protein" refers to a molecular chain of amino acids with biological activity.

The "plexin-B1" gene corresponds to the gene called "plexin-B1" in English and has GeneBank code number ACCB007867.

The terms "peptide" and "polypeptide" refer to a protein fragment. The terms "protein" and "peptide" are indistinctly used.

The phrase "reduced expression" means that the measured gene or protein levels in cancer patients are lower than the levels measured in a control population of subjects with no history of cancer.

The term "sensitivity" refers to the detection of false negatives (negative diagnosis of cancer when the patient has cancer); 100% sensitivity means that there are no false negatives.

The term "specificity" refers to the detection of false positives (positive diagnosis of cancer when the patient does not have cancer); 100% specificity means that there are no false positives.

The term "antibody" refers to a glycoprotein exhibiting specific binding activity to a particular protein, which is called "antigen". The term "antibody" comprises monoclonal antibodies, or polyclonal antibodies, either intact or fragments thereof, and includes human, humanized and non-human origin antibodies. "Monoclonal antibodies" are homogenous populations of highly specific antibodies directed against a single site or antigenic "determinant". "Polyclonal antibodies" include heterogeneous populations of antibodies directed against different antigenic determinants.

The term "epitope", as it is used in the present invention, refers to an antigenic determinant of a protein, which is the amino acid sequence of the protein recognized by a specific antibody.

The term "solid phase", as it is used in the present invention, refers to a nonaqueous matrix to which the antibody can bind. Examples of solid phase materials include glass, polysaccharides, for example agarose, polyacrylamide, polystyrene, polyvinyl alcohol and silicones. Examples of solid phase forms are the well of an assay plate or a purification column.

The term "oligonucleotide primer", as it is used in the present invention, refers to a nucleotide sequence that is complementary of a nucleotide sequence of the *plexin-B1* gene. Each primer hybridizes with its target nucleotide sequence and acts as a DNA polymerization initiation site.

The term "probe", as it is used in the present invention, refers to a nucleotide sequence that is complementary of a nucleotide sequence derived from the *plexin-B1* gene, which can be used to detect that nucleotide sequence derived from the *plexin-B1* gene.

The term "therapeutic target" refers to nucleotide or peptide sequences against which a therapeutic compound or drug can be designed and clinically applied.

The term "agonist" refers to any molecule mimicking the biological activity of the agonized molecule. Examples of agonist molecules include proteins, peptides, natural peptide sequence variations and small organic molecules (molecular weight lower than 500 daltons), among others.

The term "recombinant expression vector" refers to a replicon to which another nucleotide sequence is bound so that this other sequence can be transported, introduced and expressed inside the cells.

The term "replicon" refers to a nucleotide sequence able to self-replicate inside cells.

The present invention is based on the discovery that both the gene expression of the *plexin-B1* gene and the concentration of the plexin-B1 protein are repressed with the development of renal cancers.

In this sense, the present invention first of all provides an *in vitro* method that comprises:

a) the detection and/or quantification of the Plexin-B 1 protein, of the mRNA of the *plexin-B1* gene, or of the corresponding cDNA in a sample of an individual, and

b) the comparison of the amount of Plexin-B 1 protein, of the amount *of plexin-B1* gene mRNA or of the amount of the corresponding cDNA detected in a sample from an individual, with their normal reference values.

Said *in vitro* method is employed to detect the presence of renal cancer in an individual, to determine the stage or severity of this cancer in an individual or to monitor the effect of the therapy administered to the individual with this cancer.

The method provided by the present invention is highly sensitive and specific and is based on subjects or individuals diagnosed with kidney cancer, having low levels of *plexin-B1* gene transcribed mRNA, reduced *plexin-B1* gene expression levels or reduced concentrations of the protein encoded by the *plexin-B1* gene (the Plexin-B protein), in comparison with the corresponding levels in samples from subjects with no clinical history of cancer, such as renal cancer for example.

The present method comprises a step of obtaining the sample from the individual. Different fluid samples, such as urine, blood, plasma, serum, pleural fluid, ascitic fluid, synovial fluid, bile, semen or cerebrospinal fluid, for example, can be worked with. The sample can also be a tissue, such as kidney tissue, for example, which can be obtained by any conventional method, preferably nephrectomy. Samples can be obtained from subjects previously diagnosed or not diagnosed with renal cancer, or also from a subject undergoing treatment, or who has previously been treated for renal cancer.

The present method also comprises a step for extracting the sample, either for obtaining the protein extract of the sample or for obtaining the total RNA extract. One of these two extracts represents the working material for the next phase. Total protein or RNA extraction protocols are well known by a person skilled in the art (Chomczynski P. et al., Anal. Biochem., 1987, 162: 156; Chomczynski P., Biotechniques, 1993, 15: 532). Any conventional assay can be used within the framework of the invention for detecting renal cancer, as long as they perform *the in vitro* measurement of *plexin-B1* gene transcribed mRNA or its complementary cDNA or the Plexin-B protein concentration in samples taken from the individuals to be analyzed and from control individuals.

Therefore, this invention provides a method to detect the presence of renal cancer in an individual, to determine the stage or severity of this cancer in an individual, or to monitor the effect of the therapy administered to an individual who presents this cancer, based either on measuring the levels of the Plexin-B protein or on measuring the level of expression of the *Plexin-B1* gene.

In the event that the protein is to be detected, specifically the Plexin-B1 protein, the method of the invention comprises a first step in which the protein extract of the sample is placed in contact with a composition of one or more specific antibodies against one or more epitopes of the Plexin-B 1 protein, and a second step, in which the complexes formed by the antibodies of the Plexin-B1 protein are quantified.

There is a wide variety of immunological assays available for detecting and quantifying the formation of specific antigen-antibody complexes; numerous competitive and non-competitive protein binding assays have been previously disclosed, and a large number of these assays are available on the market. Therefore, the Plexin-B 1 protein can be quantified with antibodies such as, for example: monoclonal antibodies, polyclonal antibodies, either intact or recombinant fragments thereof, combined antibodies and Fab or scFv antibody fragments, specific against the Plexin-B protein; these antibodies being human, humanized or of a non-human origin. There are antibodies specifically binding to the Plexin-B 1 protein which are available on the market. The antibodies used in these assays may be marked or not; the unmarked antibodies can be used in agglutination assays; the marked antibodies can be used in a wide variety of assays. The marker molecules which can be used for marking the antibodies include radionucleotides, enzymes, fluorophores, chemiluminescent reagents, enzyme substrates or cofactors, enzyme inhibitors, particles, dyes and derivatives. There is a wide variety of well known assays which can be used in the present invention using unmarked antibodies (primary antibody) and marked antibodies (secondary antibody); included among these techniques are Western-blot, ELISA (Enzyme-Linked immunosorbent assay), RIA (Radioimmunoassay), competitive EIA (Competitive enzyme immunoassay), DAS-ELISA (Double antibody sandwich-ELISA), immunocytochemical and immunohistochemical techniques, techniques based on the use of protein biochips or microarrays including specific antibodies or assays based on colloidal precipitation in formats such as dipsticks. Other ways of detecting and quantifying the Plexin-B 1 protein include affinity chromatography techniques, ligand binding assays or lectin binding assays. The preferred immunoassay in the method of the invention is a double antibody sandwich ELISA assay. Any antibody or combination of antibodies specific against one or more epitopes of the Plexin-B protein can be used in this immunoassay. As an example of one of the many possible formats of this assay, a monoclonal or a polyclonal antibody, or a fragment of this antibody, or a combination of antibodies, coating a solid phase are placed in contact with the sample to be analyzed and are incubated for a time and under conditions which are suitable for forming the antigen-antibody complexes. An indicator reagent comprising a monoclonal or polyclonal antibody, or a fragment of this antibody, or a combination of these antibodies, bound to a signal generating compound is incubated with the antigen-antibody complexes for a suitable time and under suitable conditions after washing under suitable conditions for eliminating the non-specific complexes. The presence of the Plexin-B protein in the sample to be analyzed is detected and quantified, in the event that it exists, by measuring the generated signal. The amount of Plexin-B1 protein present in the sample to be analyzed is proportional to that signal.

In the event that the mRNA or cDNA corresponding to the *plexin-Bl* gene and not the protein is to be detected, the method of the invention has also several different steps. Therefore, once the sample has been obtained and the total RNA has been extracted, the method of the invention, the detection of mRNA or of the corresponding cDNA of the *plexin-B1* gene, comprises a first step of amplification of the total RNA extract or of the corresponding cDNA synthesized by reverse transcription of the mRNA, and a second step of quantification of the amplification product of the *plexin-B1* gene mRNA or cDNA. One example of mRNA amplification consists of reverse transcribing (RT) the mRNA into cDNA, followed by the Polymerase Chain Reaction (PCR) using oligonucleotide primers, the sequences of the primers used being SEQ ID NO.1 and SEQ ID NO.2. PCR is an amplification technique for amplifying a determined nucleotide sequence (target) contained in a mixture of nucleotide sequences. An excess pair of oligonucleotide primers is used in PCR, which hybridize with the complementary strands of the target nucleotide sequence. Then, an enzyme with polymerase activity (Taq DNA Polymerase) extends each primer, using the target nucleotide sequence as a mold. The products of the extension are then converted into target sequences after the disassociation of the original target strand. New primer molecules hybridize, and the polymerase extends them; the cycle is repeated in order to exponentially increase the number of target sequences. This technique is disclosed in US patents 4,683,195 and 4,683,202. Many methods for detecting and quantifying the PCR amplification products have been previously disclosed, any of which methods could be used in this invention. In a preferred method of the invention, the amplified product is detected by electrophoresis in agarose gel in the following manner: five microliters of the amplification product are subjected to an electrophoresis separation in agarose gel at a 2% concentration in a 0.5x TBE buffer at 100 vdc for one hour. After electrophoresis, the gel is stained with ethidium bromide and the amplification product can be visualized by illuminating the gel with ultraviolet (UV) light; as an alternative to staining, and a preferred embodiment, the amplified product can be blotted onto a nylon membrane by means of Southern blotting techniques, to be detected with a specific probe of the cDNA of the suitably marked *plexin-B1* gene. In another example, mRNA detection is carried out by blotting the mRNA onto a nylon membrane by means of blotting techniques, such as the Northern blot, for example, and detecting it with specific probes of the mRNA or of the corresponding cDNA of the *plexin-B1* gene. In a particular embodiment, the amplification and quantification of the mRNA corresponding to the *plexin-B1* gene is carried out by means of real time quantitative RT-PCR (Q-PCR).

The final step of the method of the invention consists of comparing the amount of Plexin-B 1 protein, the amount of *plexin-B1* gene mRNA or the amount of the corresponding cDNA detected in a sample from an individual, with the amount of Plexin-B1 protein, the amount of *plexin-B1* gene mRNA or the amount of the corresponding cDNA detected in samples from control subjects or in previous samples from the same individual, or with normal reference values.

The method also provides an *in vitro* method for identifying and assessing the efficacy of therapeutic agents for renal cancer therapy comprising:

a) placing an immortalized kidney cell culture, in contact with the candidate agent under the conditions and for the time suitable for allowing them to interact,
b) detecting and quantifying the *plexin-B1* gene or the Plexin-B1 protein expression levels, and
c) comparing said expression levels with those of immortalized kidney cell control cultures not treated with the candidate compound.

The quantification of the *plexin-B1* gene or the Plexin-B1 protein expression levels is carried out in a manner similar to that indicated in the method of the invention for detecting *in vitro* the presence of kidney cancer in an individual.

When an agent increases the *plexin-B1* gene expression levels or reverts the effects of the reduced expression of said gene, preferably decreasing cell proliferation levels, this agent becomes a candidate for cancer therapy, and especially for renal carcinoma.

Another aspect of the invention refers to the use of nucleotide or peptide sequences derived from the *plexin-B1* gene for the search, identification, development and assesment of the efficacy of compounds for renal cancer therapy. It is noteworthy, the recent importance given to screening methods based on the competitive or non-competitive binding of the potential therapeutic molecule to therapeutic target.

The application also describes agents that induce Plexin-B1 protein expression and/or activity, or that in inhibit the effects of the repression of Plexin-B 1 protein expression. These agents, which can be identified and assessed according to the present invention, can be selected from the group formed by:

a) recombinant expression vectors expressing the Plexin-B1 protein. The vector can be introduced *in vivo* into the cells of the individual diagnosed with cancer; the expression vector can also be introduced into the cells *ex vivo,* yielding transformed cells which are subsequently introduced into the individual. Examples of recombinant expression vectors are chimeric viruses; examples of viral vectors useful for gene therapy include those vectors derived from adenovirus, herpes virus, vaccinia or any other virus whose genetic material is RNA, such as avian or murine retroviruses. In these vectors, the *plexin-B1* gene can be bound to a specific gene expression promoter of the tissue affected by the cancer, for example, the kidney, such that the expression of the vector in other tissues where it is not necessary is prevented. Other examples of systems for introducing the *plexin-B1* into the interior of tumor cells by means of a vehicle are colloidal dispersion systems; examples of colloidal dispersion systems include macromolecular complexes, nanocapsules, microspheres, beads and lipid systems such as oil-in-water emulsions, mycelia, mixed mycelia and liposomes.
b) cytotoxic agents such as toxins, molecules with radioactive atoms, or chemotherapeutic agents, included among which are, with no limit, small organic and inorganic molecules, peptides, phosphopeptides, anti-sense molecules, ribozymes, triple helix molecules, etc., inhibiting the carcinogenic effects of Plexin-B 1 protein expression and/or activity repression, and
c) Plexin-B protein agonist compounds which induce, mimic or replace one or more of the functions of the Plexin-B 1 protein.

A further aspect the invention is constituted of the use of the agents characterized in that they induce Plexin-B protein expression and/or activity, as well as the use of the Plexin-B 1 protein itself, in treating renal cancer. A preferred agent is the Plexin-B 1 protein itself. These agents, in particular the Plexin-B1 protein, may be used in the form of pharmaceutically acceptable salts, derivatives or prodrugs. These terms refer to any pharmaceutically acceptable salt, ester, solvate, hydrate or any other compound which, upon administration to the recipient is capable of providing (directly or indirectly) a compound as describe herein. However, it will be appreciated that non-pharmaceutically acceptable salts also fall within the scope of the invention since those may be useful in the preparation of pharmaceutically acceptable salts. The preparation of salts, prodrugs and derivatives can be carried out by methods known in the art.

Also constituting an aspect of the present invention is a pharmaceutical composition comprising a therapeutically effective amount of the Plexin-B 1 protein itself, along with one or more excipients and/ transporting substances. Furthermore, said composition can contain any other active ingredient inducing, mimicking or replacing one or more of the functions of the Plexin-B 1 protein. The excipients, transporting substances and auxiliary substances must be pharmaceutically and pharmacologically tolerable, so that they can be combined with other components of the formulation or preparation and do not cause adverse effects in the treated organism. The pharmaceutical compositions or formulations include those which are suitable for oral or parenteral (including subcutaneous, intradermal, intramuscular or intravenous) administration, although the best administration route depends on the condition of the patient. The formulations can be in the form of single doses. The formulations are prepared according to methods known in the pharmacology field. The active substance amounts to administer may vary according to the particularities of the therapy.

An additional aspect of this invention is the use of nucleotide or peptide sequences derived from and characteristic for the *plexin-B1* gene as targets or tools for developing drugs for treating renal cancer in an individual.

A final aspect of the present invention relates to a kit for carrying out the present invention. Thus, a kit is described that comprises an antibody that specifically recognizes the Plexin-B1 protein and a carrier in suitable packing. In another embodiment the kit of the invention comprises a primer pair designed to specifically amplify a nucleic acid having a sequence that is specific to the *plexin-B1* gene. The sequence of said primer pair is selected from SEQ ID NO: 1 and SEQ ID NO: 2. These kits can be employed to detect the presence of renal cancer in an individual, to determine the stage or severity of this cancer in an individual or to monitor the effect of the therapy administered to the individual with this cancer.

The following examples illustrate the invention.

**Example 1.- Differential analysis of *plexin-B1* gene expression in kidney tissue samples using the *Human Genome U95 DNA arrays* microarrays.**

**1.1. Materials and methods**

**Microarrays.** The microarrays used were the *GeneChip Test 3* microarray (Affymetrix, Santa Clara), which allow testing the quality of the RNA, prior to the expression analysis with the *GeneChip Human Genome U95A* array (Affymetrix, Santa Clara), representing 12,000 complete sequences of recorded genes; the *plexin-B1* gene (*plexin B1*) is represented in the microarray by the 33783_at probe set of Affymetrix, which are *sense* oligonucleotides of 25 nucleotides in length, designed on the basis for the Hs.200480 sequence ofUnigene, or AB007867 of GeneBank (Table 1).

Table 1. Description of the probes corresponding to the 33783_at probe set

| Consecutive order of the probes | Zone of the interrogated reference sequence | Sequence of the probe (5'→3') | Probe position in mRNA sequence |
|---|---|---|---|
| 1 | 6720 | SEQ ID NO: 3 | 6508 |
| 2 | 6757 | SEQ ID NO: 4 | 6545 |
| 3 | 6775 | SEQ ID NO: 5 | 6563 |
| 4 | 6777 | SEQ ID NO: 6 | 6565 |
| 5 | 6863 | SEQ ID NO: 7 | 6651 |
| 6 | 6871 | SEQ ID NO: 8 | 6659 |
| 7 | 6882 | SEQ ID NO: 9 | 6670 |
| 8 | 6916 | SEQ ID NO: 10 | 6704 |
| 9 | 6918 | SEQ ID NO: 11 | 6706 |
| 10 | 7021 | SEQ ID NO: 12 | 6809 |
| 11 | 7024 | SEQ ID NO: 13 | 6812 |
| 12 | 7055 | SEQ ID NO: 14 | 6843 |
| 13 | 7057 | SEQ ID NO: 15 | 6845 |
| 14 | 7210 | SEQ ID NO: 16 | 6997 |
| 15 | 7222 | SEQ ID NO: 17 | 7009 |
| 16 | 7274 | SEQ ID NO: 18 | 7061 |

**Samples.** The kidney samples studied were from biopsies, obtained by surgical resection, of 7 individuals with renal oncocytoma (n=3), conventional renal carcinoma (n=3), chromophobe renal carcinoma (n=1). Neoplastic tissue and, as a negative control, non-neoplastic tissue were collected from each individual. All the samples were histologically classified in the Pathological Anatomy of the Hospital Universitario Marqués de Valdecilla, the same hospital where the samples had been collected, following the precepts of the Declaration of Helsinki. The samples were frozen in liquid nitrogen immediately after extraction thereof and were kept at -80°C until they were analyzed.

## *Genechip* gene expression analysis

The analysis was carried out with total RNA from) equimolar mixtures (pools) of total RNA from a set of 3 neoplastic kidney tissue samples from 3 individuals with renal oncocytoma (pool 1), equimolar mixtures of total RNA from a set of 3 neoplastic kidney tissue samples from 3 individuals with conventional renal carcinoma (Pool 2), total RNA from 1 neoplastic kidney tissue sample from 1 individual with chromophobe renal carcinoma (Sample #1), and, as a negative control, with equimolar mixtures of total RNA from a set (n=5) of non-neoplastic kidney tissue samples from individuates with renal oncocytoma, conventional renal carcinoma and chromophobe renal carcinoma (Pool 3) (Table 2).

Table 2. Description of the kidney tissue samples analyzed

|  | Renal oncocytoma | Conventional renal carcinoma | Chromophobe renal Carcinoma |
|---|---|---|---|
| Neoplastic tissue samples | Pool 1 (n=3) | Pool 2 (n=3) | #1 (n=1) |
| Non-neoplastic tissue samples | Pool 3 (n=5) |  |  |

## cRNA Synthesis

The total RNA of each one of the biopsies was obtained by homogenizing the tissue in TRIzol® Reagent (Life Technologies), following the supplier recommendations. The resulting total RNA was cleaned with the Rneasy (QIAGEN) kit (Chomczynski P. et al., Anal. Biochem., 1987, 162: 156; Chomczynski P., Biotechniques, 1993, 15: 532). 10 $\mu$g of every total RNA preparation were used as starting material for the synthesis of the first cDNA strand with the SuperScript™ II RNase reverse transcriptase enzyme (Life Technologies), using an oligo-dT oligonucleotide containing the sequence of the promoter of the RNA polymerase of the T7 phage, as a primer. The second cDNA strand was synthesized using *E. coli* DNA polymerase I enzymes (Invitrogen Life Technologies), E. *coli* DNA ligase (Invitrogen Life Technologies), *E. coli* Rnase H (Invitrogen Life Technologies), and DNA polymerase of T4 phage (Invitrogen Life Technologies). The **cRNA** marked with biotin was synthesized using the ENZO BioArray™ HighYield™ Transcript Labeling Kit (Enzo Diagnostics Inc). After the *in vitro* transcription, the unincorporated nucleotides were removed by using the Rneasy columns (QIAGEN).

## Array hybridization and scanning

15 $\mu$g of each biotinylated **cRNA** were fragmented at 94°C for 35 minutes in a buffer solution containing 40 mM Tris-Acetate (pH 8.1), 100 mM KOAc and 30 mM MgOAc. The fragmented **cRNA** was mixed with a hybridization buffer (100 mM MES, 1M NaCl, 20 mM EDTA, 0.01 % Tween 20) and was heated at 99° for 5 minutes and then at 45° for 5 minutes, to then be loaded in the Affymetrix array. The first array in which hybridization was carried out was Affymetrix Test 3. This array allows testing the quality of the RNA prior to expression analysis in the Affymetrix® Gene-Chip® *Human Genome* 95 A (HG-U95A).

For hybridization, the arrays were incubated in a rotary oven at 45° for 16 hours and with a constant rotation of 60 rpm.

Washing and staining of each array were carried out in the Affymetrix® Fluid Station. A washing and staining program was used which included:

- 10x2 washing cycles with SSPE-T 6x (0.9 m NaCl, 60 mM NaH$_2$PO4, 6 mM EDTA, 0.01 % Tween 20) at 25°,
- 4x15 cycles with 0.1 mM MES, 0.1M NaCl, 0.01% Tween 20 at 50° ,
- Biotinylated **cRNA** staining with a streptavidin-phycoerythrin conjugate (10 $\mu$g/ml Molecular Probes)
- 10x4 washing cycles with SSPE-T at 25°
- Staining with an anti-streptavidin antibody for 10 minutes
- Staining a streptavidin-phycoerythrin conjugate (1 mg/ml, Molecular Probes) for 10 minutes
- 15x4 washing cycles with SSPE-T at 30°

The arrays were scanned at 560 nm using a confocal microscope which uses laser emission (Agilent GeneArray Scanner). Intensity reading analysis was carried out with the Microarray Suite 5.0 software. To compare the arrays, they were scaled up to a total intensity of 100.

**1.2. Results.**

Differential *plexin-B1* gene expression analysis in the neoplastic tissues compared to the control, non-neoplastic tissues, was carried out starting from the array comparison data obtained using the Affymetrix software. The parameters considered (in the order in which they appear on the list) were: i) <u>Detection.</u> This indicates if the transcript is Present (P), Absent (A) or Marginal (M), ii) <u>Change:</u> This indicates if the expression of a certain transcript Increases (I), Decreases (D), experiences No Change (NC), Marginally Increases (MI), or Marginally Decreases (MD), iii) <u>Signal Log Ratio (SLR):</u> This indicates the expression change level between the baseline (control) and a test sample. This change is expressed as $\log_2$ of the ratio (fold change or number of times the expression of the gene increases or is repressed in the tumor test sample compared to the health control sample). An SLR value of 1 (equivalent to a fold change of 2) is considered significant for transcripts having an expression that increases compared to the control, and an SLR value of -1 is considered significant for transcripts having an expression that decreases compared to the control.

Differential *plexin-B1* gene expression analysis in the tumor stages with regard to the control showed that the *plexin-B1* gene expression levels were repressed in the neoplastic kidney tissue with regard to the non-neoplastic tissue, and that the greater the malignity of the analyzed tumor, the greater this repression became: the repression level was greater than 2 fold (SLR < -1) in renal oncocytoma (benign) biopsies, greater than 4 fold (SLR<-2) in conventional renal carcinoma (malignant) biopsies and greater than 8 fold (SLR<-3) in chromophobe renal carcinoma (higher degree of malignity) biopsies (Table 3).

Table 3. Results obtained for Plexin B1. Acc. N. AB007867

| Oncocytoma vs. Control | | | | |
|---|---|---|---|---|
| | | | | |
| Affy Seq | Detection Pool 3 | Detection pool1 | SLR pool 1 vs. Pool 3 | CHANGE pool 1 vs. Pool 3 |
| 33783 at | P | A | -1,2 | D |
| | | | | |
| | | | | |
| Conventional carcinoma vs. Control | | | | |
| | | | | |
| Affy Seq | Detection Pool 3 | Detection Pool 2 | SLR Pool 2 vs. Pool 3 | CHANGE Pool 2 vs. Pool 3 |
| 33783 at | P | A | -2,4 | D |
| | | | | |
| | | | | |
| Chromophobe carcinoma vs. Control | | | | |
| | | | | |
| Affy Seq | Detection Pool 3 | Detection Samples #1 | SLR #1 vs. Pool 3 | CHANGE #1 vs. Pool 3 |
| 33783 at | P | A | -3,6 | D |

**Example 2.- Differential *plexin-B1* gene expression analysis in kidney tissue samples using the quantitative real time RT-PCR technique.**

**2.1. Materials and methods**

To determine the *plexin-B1* gene expression levels, quantitative real time RT-PCR was carried out in a *7000 Sequence Detection System* using an *SYBR green PCR master mix* (Applied-Biosystems, Foster City, USA). The total RNA was extracted from individual biopsies using Trizol™ Reagent (Life Technologies, USA), following the instructions recommended by the manufacturer; this total RNA was then purified with the *Qiagen Mini Kit spin columns* (Qiagen, USA.). The integrity of the RNA was confirmed by electrophoresis in 1% agarose gel and the RNA was spectrophoto-metrically quantified. Then, 5 $\mu$g pf total RNA were digested with DNase I (1.2 uu/$\mu$g RNA) and the cDNA was synthesized following the following protocol: One $\mu$g of RNA treated with DNAse was mixed, in a total volume of 20 $\mu$l, with *Super-Script™ Rnase H-Reverse Transcriptase* (Invitrogen, USA.) (400 units/$\mu$g RNA) and with 100 pmols of oligo-dT. The synthesized cDNA was amplified using specific primers of the human *plexin-B1* gene (SEQ ID NO: 1, and SEQ ID NO: 2), and specific primers of the human glyceraldehyde-3-phosphate-dehydrogenase (gapdh) gene. Then, the ratio between the abundance of transcribed *plexin-B1* mRNAs and the abundance of the gapdh transcripts was calculated, as the relative measurement of gene expression: $2^n$, where n is the value $C_t$ (*threshold cycle*) of gapdh minus the *plexin-B1* $C_t$ value, and the data of the ratio was normalized based on the value of the sample with the lowest *plexin-B1* expression level. The specificity of the PCR products was determined by denaturation curve analysis. A standard curve was constructed for each gene sequence, carried out with serial dilutions of cDNA; the mold cDNA concentrations were given the arbitrary values 10, 5, 2.5, 1.25, 0.625, 0.3125 and 0.15625 for the reactions on the standard curve. The real time PCR reactions were prepared using the *LightCycler-FastStart DNA master SYBR Green I kit* (Roche, USA.), following the instructions of the manufacturer. The amplification program consisted of 1 cycle of 95°C for 1 min (hot start) followed by 45 cycles of 95°C (denaturation) for 10 seconds, 60°C (annealing) for 5 seconds, 72°C (amplification and acquisition) for 10 seconds. The denaturation curve analysis program consisted of one cycle of a 95°C, 65°C pulse for 15 seconds, and a 95°C pulse during the amplification and acquisition step.

Amplification efficiency was calculated for each PCR reaction based on the standard curve data, using the equation:

$$E = 10^{-1/slope} \qquad [1]$$

where E is the amplification efficiency.
The ratio of the gene expression values were determined by the equation:

$$Ratio = \frac{E_{target}^{-(Cp\ target\ control - Cp\ target\ sample)}}{E_{reference}^{-(Cp\ reference\ control - Cp\ reference\ sample)}} \qquad [2]$$

where E is the amplification efficacy, *Cp* is the cross point, *target* is *plexin-B1, reference* is GAPDH, *control* is the healthy sample and *sample* is the tumor sample.

## 2.2. Results

Amplification and denaturation curves of the target gene (*plexin-B1*) and of the reference gene (gadph) were constructed; on the denaturation curve, a single defined peak was observed at the temperature corresponding to the denaturation temperature of each product, which indicated that the reactions were specific (data not shown). Each samplewas analyzed in triplicate to calculate the relative change in the gene expression levels using equations [1] and [2] described in the previous section. The changes in the *plexin-B1* gene expression levels are shown in Figure 1. Only one out of the neoplastic samples analyzed showed gene expression levels exceeding the levels in the non-neoplastic levels; in the other cases, which included conventional, papillary and chromophobe renal carcinoma (table 4), expression levels were clearly repressed with regard to the samples from non-neoplastic tissue (Figure 1).

Table 4. Clinicopathological data of the renal neoplasms analyzed

| Case | Age | Sex | Histological Diagnosis | Stage | Evolution |
|------|-----|-----|------------------------|-------|-----------|
| 1 | 58 | Male | Conventional RC | II | A&H (28 months) |
| 2 | 80 | Male | Conventional RC | II | A&H (16 months) |
| 3 | 78 | Male | Conventional RC | II | A&H (20 months) |
| 4 | 38 | Male | Conventional RC | II | A&H (24 months) |
| 5 | 68 | Male | Conventional RC | II | A&H (24 months) |
| 6 | 76 | Female | Conventional RC | II | A&H (22 months) |
| 7 | 51 | Male | Conventional RC | III | DDD (15 months) |
| 8 | 45 | Male | Conventional RC | III | DDD (11 months) |
| 9 | 57 | Male | Chromophobe RC | II | A&H (25 months) |
| 10 | 55 | Male | Papillary RC (low grade) | II | A&H (24 months) |
| 11 | 69 | Male | Papillary RC (high grade) | III | A&H (18 months) |

(continued)

| Case | Age | Sex | Histological Diagnosis | Stage | Evolution |
|------|-----|-----|------------------------|-------|-----------|
| 12 | 79 | Male | Renal oncocytoma | II | A&H (26 months) |

RC: Renal carcinoma A&H (n months): Alive and Healthy, n months after diagnosis.

DDD (n months): Death due to disease, n months after diagnosis.

## Example 3. Proliferation assays in parental and transfected ACHN human renal adenocarcinoma cell line.

### 3.1. Materials and Methods

### Transfection and cell cloning

ACHN human renal adenocarcinoma cells were obtained from the European Collection of Cell Cultures (ECACC No. 88100508), and cultured in DMEM medium supplemented with 10 % FCS, 2 mM glutamine and antibiotics (all from Gibco/BRL, Paisley, UK). For transfection, $10^6$ cells were plated in 10-cm tissue culture dishes (Corning, Coming, USA) and transfected 24 h later with 10 $\mu$g of plasmid DNA by the calcium phosphate method. The eukaryotic expression vector pCEFL/plexinB1 which contains a neomycin resistance expression cassette, was used to drive full-length plexinB1 expression from the EF1 promoter. As a control, vector pMEX was used (Katzav S, Martin-Zanca D, Barbacid M, 1989 EMBO J, vol 8, pp. 2283-2290), which also contains the neomycin resistance cassette. 48 h after transfection, cells were transferred to 96-well plates (Corning) and 1 mg/ml Geneticin (Gibco/BRL) was added to the culture medium to select for stably transfected clones. After expansion in 75-cm$^2$ flasks (Coming), plexinB1 expression in Geneticin-resistant clones was verified by standard RT-PCR methods with primers located at the 3' end of the coding sequence (SEQ ID NO: 19 [forward] and SEQ ID NO: 20 [reverse]). As a control, RT-PCR was performed on the housekeeping gene rib 110 (SEQ ID NO: 21 [forward] and SEQ ID NO: 22 [reverse]).

### MTT (cell proliferation) assay

500 parental ACHN, ACHN/pMEX (AM) or ACHN/plexinB1 (AP) cells were seeded per well in 200 $\mu$l of culture medium in 96-well plates (Corning) and in replicates of 8. After incubation for 24, 48, 72 or 96 h, 20 $\mu$l of a stock solution of MTT (Sigma, Steinheim, Germany) was added to the cultures for a final concentration of 50 $\mu$g/ml. Upon incubation for 2 h at 37C, the medium was decanted and 200 $\mu$l DMSO were added to each well. Plates were incubated for 30 min at room temperature to allow dissolution of MTT crystals. Optical density (OD) readings were taken on a Labsystems (Ashford, UK) Multiscan RC plate reader fitted with 570 nm and 650 nm filters. Absorbance was determined by averaging the difference between $OD_{570}$ and $OD_{650}$ readings. Regression curves for cell proliferation data were calculated and plotted with Microsoft Excel 2000 software.

### 3.2 Results

All plexinB1-expressing clones (AP2-AP12) proliferate more slowly than the control clone (AM2) or parental ACHN cells (Table 5 and Figure 2) in both experiments.

Table 5. Values for the growth rate in 2 independent determinations

|  | Exp. # 1 | Exp.#2 |
|------|----------|--------|
| ACHN | - | 0.7019 |
| AM2 | 0.5346 | 0.6366 |
| AP2 | 0.3632 | 0.5581 |
| AP3 | 0.3664 | 0.5876 |
| AP4 | 0.3678 | 0.5407 |
| AP5 | 0.3179 | 0.4007 |
| AP7 | 0.3430 | 0.5509 |
| AP8 | 0.3484 | 0.4802 |

(continued)

|  | Exp. # 1 | Exp.#2 |
|---|---|---|
| AP9 | 0.2817 | 0.4400 |
| AP10 | 0.3475 | 0.5169 |
| AP11 | 0.2998 | 0.5821 |
| AP12 | 0.3047 | - |

### 3.3 Discussion

The MTT (cell proliferation) assays have shown that the proliferation rate of ACHN human renal adenocarcinoma cells is significantly lower when those cells are expressing Plexin B1. These results suggest that the expression of Plexin B1 causes a decrease in cell proliferation. These functional data, altogether with data at the molecular expression level, from DNA microarrays, Q-PCR and Inmunohistochemistry, suggest an antiproliferative role for Plexin-B 1 in renal carcinomas.

### Example 4. Analysis of protein expression in tissue samples using tissue arrays

### 4.1. Materials and Methods

Fixed paraffin-embedded tumour samples from the pathology archives of the Hospital Universitario Marqués de Valdecilla were sectioned and arrayed on glass slides. In total 51 cases of adult renal cell carcinoma [26 cases of clear cell (conventional) renal cell carcinoma and 25 cases of papillary renal cell carcinoma] from nephrectomy specimens and 6 healthy kidney samples were examined by immuno-histochemical staining. All paraffin-embedded donor tissue blocks were sampled with 1.00-mm punchers using a Beecher tissue microarray instrument (Beecher Instruments Inc. Sun Prairie, WI, USA). Paraffin tissue array blocks containing arrayed core cylinders were subjected to routine staining with hematoxylin and eosin followed by immunohistochemical staining for the Plexin B1. Briefly, antigen retrieval was performed by boiling sections in citric acid buffer in a pressure cooker for 180 sec. Next, a 1:100 dilution of a rabbit polyclonal serum raised against a KLH-plexinB1 peptide conjugate (SEQ ID NO. 23, residues 1113-1127) was incubated with the specimen. This was followed by visualization with the Dako EnVisionTM+ system (Dako, Glostrup, Denmark), which is based on the use of a secondary goat-anti-rabbit antibody conjugated to a peroxidase-linked polymer followed by a chromogen substrate (diaminobenzidine). The immunostaining process was performed on a Techmate 500-220 automated immunostainer (Biotek, Santa Barbara, CA, USA).

To reduce interobserver variability in the histopathological evaluation of the antibody-stained specimens three independent pathologists from the Pathological Anatomy Department of the University Hospital Marques de Valdecilla evaluated staining patterns and scoring criteria were agreed. Positive staining of Plexin B1 was defined as a coarse cytoplasmic membrane reactivity. Immunohistochemistry was considered negative in cases where staining was absent or which showed weak staining (<5% of cells in a given section).

### 4.2. Results

The specificity of the immunohistochemical staining is supported by the following findings: (1) Staining is tissue specific, i.e. present in healthy tissue but absent in a significant part of tumor tissues, in agreement with microarray and Q-PCR data. (2) Staining is positive for renal tubule cells, cells in which the tumors under study originate, but not for other cell types such as vascular cells, mesenchymal cells or the inflammatory infiltrate. (3) Staining is present on the cell membrane and the cytoplasm, without any precipitates, but is absent from the nucleus. (4) Staining is blocked by addition of the immunizing peptide.

Of the adult renal cell carcinoma sections that were analysed immunohistochemically a positive reaction with the antibody specific for Plexin B1 was positive in 19% of clear cell (conventional) renal cell carcinoma sections, 40% of papillary renal cell carcinoma compared to the 83% of healthy positive sample (table 6).

Table 6. Results of Immunohistochemical Staining

| | Total n° of samples | Positive cases | Negative cases | % of positive cases* |
|---|---|---|---|---|
| **clear cell (conventional) renal cell carcinoma** | 26 | 5 | 21 | **19%** |
| **papillary renal cell carcinoma** | 25 | 10 | 15 | **40%** |
| **healthy kidney samples** | 6 | 5 | 1 | **83%** |
| | | | | |
| * Positive / (Positive + Negative) x 100 | | | | |

### 4.3. Discussion

Consistent with the Plexin B1 gene expression data in renal cell carcinomas versus healthy kidney, sections classified as carcinomas showed a lower percentage of positive staining with labelled Plexin B1 antibody (19% and 40%) than sections corresponding to healthy kidney (83%). These results show that Plexin B1 protein is not expressed in a high percentage of renal cell carcinomas.

**Claims**

1. An *in vitro* method for detecting the presence of renal cancer in an individual, to determine the stage or severity of this cancer in an individual or to monitor the effect of the therapy administered to the individual with this cancer that comprises:

   a) the detection and/or quantification of the Plexin-B1 protein, of the mRNA of the *plexin-B1* gene, or of the corresponding cDNA in a sample of an individual, and
   b) the comparison of the amount of Plexin-B1 protein, of the amount of *plexin-B1* gene mRNA or of the amount of the corresponding cDNA detected in a sample from an individual, with their normal reference values.

2. Method according to claim 1, wherein said sample is a kidney tissue sample.

3. Method according to claim 2, wherein said kidney tissue sample to be analyzed is obtained by any conventional method, preferably nephrectomy.

4. Method according to claim 1, wherein said sample is a urine, blood, plasma, serum, pleural fluid, ascitic fluid, synovial fluid, bile, semen, gastric juice or cerebrospinal fluid sample.

5. Method according to claim 1, wherein said sample to be analyzed is obtained from an individual who has not previously been diagnosed with renal cancer.

6. Method according to claim 1, wherein said sample to be analyzed is obtained from an individual who has previously been diagnosed with renal cancer.

7. Method according to claim 1, wherein said sample to be analyzed is obtained from an individual undergoing treatment, or who has been treated previously, for renal cancer.

8. Method according to claim 1, **characterized in that** it comprises the extraction of the sample, either for obtaining a protein extract or for obtaining an extract of total RNA.

9. Method according to claim 1, **characterized in that** the detection and/or quantification of the Plexin-B1 protein comprises a first step, in which the protein extract of the sample is placed in contact with a composition of one or more specific antibodies against one or more epitopes of the Plexin-B1 protein, and a second step, in which the complexes formed by the antibodies and the Plexin-B 1 protein are quantified.

10. Method according to claim 9, **characterized in that** said antibodies comprise monoclonal antibodies, polyclonal antibodies, either intact or recombinant fragments thereof, combined antibodies and Fab or scFv antibody fragments, specific against the Plexin-B1 protein; these antibodies being human, humanized or of a non-human origin.

11. Method according to claims 9 or 10, **characterized in that** in the detection and/or quantification of the complexes formed by the antibodies and the Plexin-B1 protein, the techniques used are selected from the group formed by: Western-blot, ELISA (Enzyme-Linked Immunosorbent Assay), RIA (Radioimmunoassay), Competitive EIA (Competitive Enzyme Immunoassay), DAS-ELISA (Double antibody Sandwich-ELISA), immunocytochemical and immunohistochemical techniques, techniques based on the use of biochips or protein microarrays that include specific antibodies, assays based on precipitation with colloidal gold in formats such as dipsticks; or by means of affinity chromatography techniques, ligand binding assays or lectin binding assays.

12. Method according to claim 1, **characterized in that** the detection and/or quantification either of the mRNA or of the corresponding cDNA of the *plexin-B1* gene comprises a first step of amplification of the mRNA that is present in the extract of total RNA, or of the corresponding cDNA synthesized by reverse transcription of the mRNA; and a second step of quantification of the amplification product from either the mRNA or the cDNA of the *plexin-B1* gene.

13. Method according to claim 12, **characterized in that** the amplification is performed qualitatively or quantitatively by means of RT-PCR using primer oligonucleotides, where the sequences of the primers used to amplify the sequence of the *plexin-B1* gene are SEQ ID NO: 1 and SEQ ID NO: 2.

14. Method according to claim 1, **characterized in that** the detection and/or quantification is done with specific probes of the mRNA or of the corresponding cDNA of the *plexin-B1* gene by techniques such as the Northern-blot or Northern transfer.

15. Method according to claim 1, **characterized in that** the detection of the mRNA is done by real time quantitative RT-PCR (Q-PCR).

16. Use of nucleotide or peptide sequences derived from the *plexin-B1* gene to *detect in vitro* the presence of renal cancer in an individual, for determining *in vitro* the stage or severity of said cancer in the individual or for *monitoring in vitro* the effect of the therapy administered to an individual having said cancer.

17. An *in vitro* method for identifying and assessing the efficacy of compounds for renal cancer therapy, comprising:

   a) placing an immortalized kidney cell culture, in contact with the candidate agent under the conditions and for the time which are suitable for allowing them to interact,
   b) detecting and quantifying the *plexin-B1* gene or Plexin-B 1 protein expression levels, and
   c) comparing said expression levels with those of immortalized kidney cell control cultures not treated with the candidate compound.

18. Use of nucleotide or peptide sequences derived from the *plexin-B1* gene in methods for the search, identification, development and assessment of the efficacy of compounds for renal cancer therapy, wherein said use is not a method for treatment of the human or animal body by surgery or therapy or a diagnostic method practised on the human or animal body.

19. A recombinant expression vector expressing the Plexin B1 protein for treating renal cancer.

20. Use of a recombinant expression vector expressing the Plexin B1 protein in the preparation of a medicament for treating renal cancer.

21. Use of the Plexin-B1 protein in the preparation of a medicament for treating renal cancer.

22. Pharmaceutical composition comprising a therapeutically effective amount of a Plexin-B1 protein and at least one pharmaceutically acceptable excipient.

23. Pharmaceutical composition according to claim 22, **characterized in that** it contains another drug substance, preferably one inducing the Plexin-B1 protein function.

24. Use of a kit that comprises an antibody that specifically recognizes the Plexin-B1 protein and a carrier in suitable packaging to detect the presence of kidney cancer in an individual, to determine the stage or severity of this cancer in an individual, or to monitor the effect of the therapy administered to the individual with this cancer, wherein said use is not a method for treatment of the human or animal body by surgery or therapy or a diagnostic method practised on the human or animal body.

**25.** A kit comprising a primer pair designed to specifically amplify a nucleic acid having a sequence that is specific to the *plexin-B1* gene, wherein the sequence of the primer pair is selected from SEQ ID NO: 1 and SEQ ID NO: 2.

**26.** Use of a kit that comprises a primer pair designed to specifically amplify a nucleic acid having a sequence that is specific to the *plexin-B1* gene, to detect the presence of kidney cancer in an individual, to determine the stage or severity of this cancer in an individual, or to monitor the effect of the therapy administered to the individual with this cancer.

**27.** Use of a kit according to claim 26, wherein the sequence of the primer pair is selected from SEQ ID NO: 1 and SEQ ID NO: 2.

**Patentansprüche**

**1.** *In vitro*-Verfahren zum Nachweis des Vorhandenseins von Nierenkrebs in einem Individuum, um das Stadium oder den Ausprägungsgrad dieses Krebses in einem Individuum zu bestimmen oder um den Effekt der Therapie zu verfolgen, die dem Individuum mit diesem Krebs verabreicht wurde, umfassend:

a) den Nachweis und/oder die Quantifizierung des Plexin-B1-Proteins, der mRNA des *plexin-B1*-Gens oder der entsprechenden cDNA in einer Probe eines Individuums, und
b) den Vergleich der Menge an Plexin-B1-Protein, der Menge an *plexin-B1*-Gen-mRNA oder der Menge der entsprechenden cDNA, die in einer Probe des Individuums nachgewiesen wurde, mit ihren normalen Referenzwerten.

**2.** Verfahren nach Anspruch 1, wobei die Probe eine Nierengewebeprobe ist.

**3.** Verfahren nach Anspruch 2, wobei die zu analysierende Nierengewebeprobe durch ein beliebiges konventionelles Verfahren erhalten wurde, vorzugsweise durch Nephrektomie.

**4.** Verfahren nach Anspruch 1, wobei die Probe eine Urin-, Blut-, Plasma-, Serum-, Pleuraerguss-, Aszitesflüssigkeits-, Synovialflüssigkeits-, Gallen-, Samen-, Magensaftoder Cerebrospinalflüssigkeitsprobe ist.

**5.** Verfahren nach Anspruch 1, wobei die zu analysierende Probe aus einem Individuum erhalten wurde, bei dem zuvor kein Nierenkrebs diagnostiziert wurde.

**6.** Verfahren nach Anspruch 1, wobei die zu analysierende Probe aus einem Individuum erhalten wurde, bei dem zuvor Nierenkrebs diagnostiziert wurde.

**7.** Verfahren nach Anspruch 1, wobei die zu analysierende Probe aus einem Individuum erhalten wurde, das sich wegen Nierenkrebs in Behandlung befindet oder das zuvor wegen Nierenkrebs behandelt wurde.

**8.** Verfahren nach Anspruch 1, **dadurch gekennzeichnet, dass** es eine Extraktion der Probe umfasst, entweder um einen Proteinextrakt zu erhalten oder um ein Extrakt der Gesamt-RNA zu erhalten.

**9.** Verfahren nach Anspruch 1, **dadurch gekennzeichnet, dass** der Nachweis und/oder die Quantifizierung des Plexin-B1-Proteins einen ersten Schritt umfasst, in dem der Proteinextrakt der Probe mit einer Zusammensetzung von einem oder mehreren spezifischen Antikörpern in Kontakt gebracht wird, die gegen ein oder mehrere Epitope des Plexin-B1-Proteins gerichtet sind, und einen zweiten Schritt umfasst, in dem die Komplexe, die durch die Antikörper und das Plexin-B1-Protein gebildet wurden, quantifiziert werden.

**10.** Verfahren nach Anspruch 9, **dadurch gekennzeichnet, dass** die Antikörper monoclonale Antikörper, polyclonale Antikörper, entweder intakte oder rekombinante Fragmente davon, kombinierte Antikörper und Fab- oder scFv-Antikörperfragmente umfassen, die spezifisch gegen das Plexin-B1-Protein gerichtet sind; wobei diese Antikörper menschliche, humanisierte oder nicht-menschlichen Ursprungs sind.

**11.** Verfahren nach Anspruch 9 oder 10, **dadurch gekennzeichnet, dass** bei dem Nachweis und/oder der Quantifizierung der Komplexe, die durch die Antikörper und das Plexin-B1-Protein gebildet wurden, die verwendeten Methoden ausgewählt sind aus der Gruppe bestehend aus: Western-Blot, ELISA (Enzymverbundener Immunadsorptions-Assay), RIA (Radioimmunassay), Kompetitiver EIA (Kompetitiver Enzymimmunassay), DAS-ELISA (Doppel-Antikörper-Sandwich-ELISA), immuncytochemische und immunhistochemische Methoden, Methoden, die auf der Verwendung von Biochips oder Protein-Microarrays basieren, die spezifische Antikörper beinhalten, Tests, die auf der Ausfällung mit kolloidalem Gold in Formaten wie zum Beispiel Teststäbchen basieren; oder durch Affinitätschromatographie-Verfahren, Ligandbindungstests oder Lectinbindungstests.

**12.** Verfahren nach Anspruch 1, **dadurch gekennzeichnet, dass** der Nachweis und/oder die Quantifizierung entweder der mRNA oder der entsprechenden cDNA des *plexin-B1*-Gens einen ersten Schritt der Amplifikation der mRNA umfasst, die in dem Extrakt der Gesamt-RNA vorhanden ist, oder der entsprechenden cDNA, die durch reverse Transkription der mRNA synthetisiert wurde; und einen zweiten Schritt der Quantifizierung des Amplifikationsprodukts entweder der mRNA oder der cDNA des *plexin-B1*-Gens umfasst.

**13.** Verfahren nach Anspruch 12, **dadurch gekennzeichnet, dass** die Amplifikation qualitativ oder quantitativ durch RT-PCR durchgeführt wird unter Verwendung von Primer-Oligonucleotiden, wobei die Sequenzen der Primer, die verwendet werden, um die Sequenz des *plexin-B1*-Gens zu amplifizieren, SEQ ID NO:1 und SEQ ID NO:2 sind.

**14.** Verfahren nach Anspruch 1, **dadurch gekennzeichnet, dass** der Nachweis und/oder die Quantifizierung mit spezifischen Sonden der mRNA oder der entsprechenden cDNA des *plexin-B1*-Gens durch Methoden wie Northern-Blot oder Northern-Transfer durchgeführt wird.

**15.** Verfahren nach Anspruch 1, **dadurch gekennzeichnet, dass** der Nachweis der mRNA durch quantitative Echtzeit-RT-PCR (Q-PCR) durchgeführt wird.

**16.** Verwendung von Nucleotid- oder Peptidsequenzen, welche von dem *plexin-B1*-Gen abgeleitet sind, um *in vitro* das Vorhandensein von Nierenkrebs in einem Individuum nachzuweisen, um *in vitro* das Stadium oder den Ausprägungsgrad dieses Krebses in einem Individuum zu bestimmen oder um *in vitro* den Effekt der Therapie zu verfolgen, die dem Individuum, welches den Krebs hat, verabreicht wurde.

**17.** *In vitro*-Verfahren zur Identifizierung und Beurteilung der Wirksamkeit von Verbindungen für die Nierenkrebstherapie, umfassend:

   a) Inkontaktbringen einer immortalisierten Nierenzellkultur mit einem Wirkstoffkandidaten unter den Bedingungen und für die Zeit, die geeignet sind, deren Interaktion zu ermöglichen,
   b) Nachweisen und Quantifizieren des *plexin-B1*-Genoder Plexin-B1-Protein-Expressionsspiegels, und
   c) Vergleichen der Expressionsspiegel mit denen von immortalisierten Nierenzellen-Kontrollkulturen, die nicht mit dem Wirkstoffkandidaten behandelt wurden.

**18.** Verwendung von Nucleotid- oder Peptidsequenzen, die von dem *plexin-B1*-Gen abgeleitet sind, in Verfahren für die Suche, Identifizierung, Entwicklung und Beurteilung der Wirksamkeit von Verbindungen für die Nierenkrebstherapie, wobei die Verwendung kein Verfahren zur chirurgischen oder therapeutischen Behandlung des menschlichen oder tierischen Körpers oder kein Diagnostizierverfahren ist, das am menschlichen oder tierischen Körper vorgenommen wird.

**19.** Rekombinanter Expressionsvektor, der das Plexin-B1-Protein exprimiert, zur Behandlung von Nierenkrebs.

**20.** Verwendung eines rekombinanten Expressionsvektors, der das Plexin-B1-Protein exprimiert, zur Herstellung eines Arzneimittels für die Behandlung von Nierenkrebs.

**21.** Verwendung des Plexin-B1-Proteins zur Herstellung eines Arzneimittels für die Behandlung von Nierenkrebs.

**22.** Arzneimittel, umfassend eine therapeutisch wirksame Menge eines Plexin-B1-Proteins und mindestens einen pharmazeutisch verträglichen Exzipienten.

**23.** Arzneimittel nach Anspruch 22, **dadurch gekennzeichnet, dass** es einen anderen Arzneistoff enthält, vorzugsweise einen, der die Plexin-B1-Proteinfunktion induziert.

**24.** Verwendung eines Kits, das einen Antikörper, der spezifisch das Plexin-B1-Protein erkennt, und einen Träger umfasst, in geeigneter Verpackung, um das Vorhandensein von Nierenkrebs in einem Individuum nachzuweisen, um das Stadium oder den Ausprägungsgrad dieses Krebses in einem Individuum zu bestimmen oder um den Effekt der Therapie zu verfolgen, die dem Individuum mit diesem Krebs verabreicht wurde, wobei die Verwendung kein Verfahren zur chirurgischen oder therapeutischen Behandlung des menschlichen oder tierischen Körpers oder kein Diagnostizierverfahren ist, das am menschlichen oder tierischen Körper vorgenommen wird.

**25.** Kit, umfassend ein Primerpaar, das entworfen wurde, um speziell eine Nucleinsäure zu amplifizieren, die eine Sequenz hat, die spezifisch für das *plexin-B1*-Gen ist, wobei die Sequenz des Primerpaars ausgewählt ist aus der Gruppe bestehend aus SEQ ID NO:1 und SEQ ID NO:2.

**26.** Verwendung eines Kits, das ein Primerpaar umfasst, das entworfen wurde, um speziell eine Nucleinsäure zu amplifizieren, die eine Sequenz hat, die spezifisch für das *plexin-B1*-Gen ist, um das Vorhandensein von Nierenkrebs in einem Individuum nachzuweisen, das Stadium oder den Ausprägungsgrad dieses Krebses in einem Individuum zu bestimmen oder um den Effekt der Therapie zu verfolgen, die dem Individuum mit diesem Krebs verabreicht wurde.

**27.** Verwendung eines Kits nach Anspruch 26, wobei die Sequenz des Primerpaars ausgewählt ist aus SEQ ID NO:1 und SEQ ID NO:2.

## Revendications

**1.** Méthode *in vitro* pour détecter la présence d'un cancer du rein chez un individu pour déterminer le stade ou la sévérité de ce cancer chez un individu ou surveiller l'effet de la thérapie administrée à l'individu ayant ce cancer et qui comprend :

a) la détection et/ou la quantification de la protéine Plexine-B1, de l'ARNm du gène *plexine-B1*, ou de l'ADNc correspondant dans un prélèvement d'un individu, et
b) la comparaison de la quantité de protéine Plexine-B1, de la quantité d'ARNm du gène *plexine-B1* ou de la quantité de l'ADNccorrespondant détecté dans un prélèvement provenant d'un individu, avec leurs valeurs normales de référence.

**2.** Méthode selon la revendication 1, **caractérisé en ce que** le dit prélèvement est un prélèvement de tissu rénal.

**3.** Méthode selon la revendication 2, **caractérisée en ce que** le dit prélèvement de tissu rénal à analyser est obtenu par toute méthode conventionnelle, de préférence par néphrectomie.

**4.** Méthode selon la revendication 1, **caractérisée en ce que** le dit prélèvement est un prélèvement d'urine, de sang, de plasma, de sérum, de liquide pleural, de liquide d'ascite, de liquide synovial, de bile, de sperme, de suc gastrique ou de liquide céphalorachidien.

**5.** Méthode selon la revendication 1, **caractérisée en ce que** le dit prélèvement à analyser est obtenu auprès d'un individu qui n'a pas été auparavant diagnostiqué avec un cancer du rein.

**6.** Méthode selon la revendication 1, **caractérisée en ce que** le dit prélèvement à analyser est obtenu auprès d'un individu qui a été auparavant diagnostiqué avec un cancer du rein.

**7.** Méthode selon la revendication 1, **caractérisée en ce que** le dit prélèvement à analyser est obtenu auprès d'un individu en cours de traitement, ou ayant été traité auparavant pour un cancer du rein.

**8.** Méthode selon la revendication 1, **caractérisée en ce qu'**elle comprend l'extraction du prélèvement, soit pour obtenir un extrait protéique ou pour obtenir un extrait d'ARN total.

**9.** Méthode selon la revendication 1, **caractérisée en ce que** la détection et/ou la quantification de la protéine Plexine-B1 comporte une première étape, dans laquelle l'extrait protéique du prélèvement est mis en contact avec une composition d'un ou plusieurs anticorps spécifiques contre un ou plusieurs épitopes de la protéine Plexine-B1, et une deuxième étape, dans laquelle les complexes formés par les anticorps et la protéine Plexine-B1 sont quantifiés.

**10.** Méthode selon la revendication 9, **caractérisée en ce que** les dits anticorps comprennent des anticorps monoclonaux, des anticorps polyclonaux, également des fragments intacts ou recombinants de ceux-ci, des anticorps combinés et des fragments d'anticorps Fab ou scFv spécifiques à la protéine Plexine-B1, ces anticorps étant humains, humanisés ou d'origine non-humaine.

**11.** Méthode selon la revendication 9 ou 10, **caractérisée en ce que** dans la détection et/ou la quantification des complexes formés par les anticorps et la protéine Plexine-B1, les techniques utilisées sont choisies dans le groupe constitué de : Western-blot, ELISA (Titrage immuno-enzymatique utilisant un antigène absorbé), RIA (Radio-immuno-étalonnage), EIA Compétitive (Titrage immuno-enzymatique utilisant un antigène absorbé Compétitif), DAS-ELISA (ELISA sandwich à double anticorps), des techniques immunocytochimiques et immunohistochimiques, des techniques basées sur l'utilisation de biopuces ou de micropuces de protéines qui incluent des anticorps spécifiques, des dosages basés sur la précipitation avec de l'or colloïdal dans des formats tels que des bandelettes réactives, ou par des techniques de chromatographie à affinité, des dosages de liaison à un ligand ou des dosages de liaison à la lectine.

**12.** Méthode selon la revendication 1, **caractérisée en ce que** la détection et/ou la quantification de l'ARNm ou de l'ADNccorrespondant au gène *plexine-B1* comprend une première étape d'amplification de l'ARNm qui est présent dans l'extrait d'ARN total, ou de l'ADNc correspondant synthétisé par la rétro-transcription de l'ARNm, et une deuxième étape de quantification du produit de l'amplification issu aussi bien de l'ARNm ou de l'ADNc du gène *plexine-B1.*

**13.** Méthode selon la revendication 12, **caractérisée en ce que** l'amplification est réalisée qualitativement ou quantitativement par RT-PCR en utilisant des amorces oligonucléotidiques, où les séquences des amorces utilisées pour amplifier la séquence du gène *plexine-B1* sont la SEQ ID NO: 1 et la SEQ ID NO: 2.

**14.** Méthode selon la revendication 1, **caractérisée en ce que** la détection et/ou quantification est faite avec des sondes spécifiques à l'ARNm ou de l'ADNc correspondant au gène *plexine-B1* par des techniques telles que le Northern-blot ou le Northern transfert.

**15.** Méthode selon la revendication 1, **caractérisée en ce que** la détection de l'ARNm est faite par une RT-PCR (Q-PCR) quantitative en temps réel.

**16.** Utilisation de séquences nucléotidiques ou peptidiques dérivées du gène *plexine-B1* pour détecter in *vitro* la présence d'un cancer du rein chez un individu, pour déterminer *in vitro* le stade ou la sévérité du dit cancer chez l'individu ou pour surveiller *in vitro* l'effet de la thérapie administrée à un individu ayant ce dit cancer.

**17.** Méthode *in* vitro pour identifier et estimer l'efficacité de composés pour le traitement du cancer du rein, comprenant :

a) le placement d'une culture de cellules rénales immortalisées en contact avec l'agent candidat dans les conditions et pour la durée appropriées pour leur permettre d'interagir,
b) la détection et la quantification des niveaux d'expression du gène *plexine-B1* ou de la protéine Plexine-B1, et
c) la comparaison des dits niveaux d'expression avec ceux des cultures de cellules rénales immortalisées de contrôle non traitées avec le composé candidat.

**18.** Utilisation de séquences nucléotidiques ou peptidiques dérivées du gène plexine-B1 dans des méthodes de recherche, d'identification, de développement et d'évaluation de l'efficacité de composés pour le traitement du cancer du rein, **caractérisée en ce que** la dite utilisation n'est pas une méthode de traitement du corps humain ou animal par chirurgie ou thérapie ou une méthode de diagnostic pratiquée sur le corps humain ou animal.

**19.** Vecteur d'expression recombinant exprimant la protéine Plexine B1 pour traiter le cancer du rein.

**20.** Utilisation d'un vecteur d'expression recombinant exprimant la protéine Plexine B1 dans la préparation d'un médicament pour traiter le cancer du rein.

**21.** Utilisation de la protéine Plexine-B1 dans la préparation d'un médicament pour le traitement du cancer du rein.

**22.** Composition pharmaceutique comprenant une quantité thérapeutiquement efficace d'une protéine Plexine-B1 et au moins un excipient pharmaceutiquement acceptable.

**23.** Composition pharmaceutique selon la revendication 22, **caractérisée en ce qu'**elle contient une autre substance médicamenteuse, de préférence une induisant la fonction de la protéine Plexine-B1.

**24.** Utilisation d'un kit comprenant un anticorps qui reconnaît spécifiquement la protéine B1 et un porteur dans un emballage approprié pour détecter la présence d'un cancer du rein chez un individu, pour déterminer le stade ou la sévérité de ce cancer chez un individu, ou pour surveiller l'effet du traitement administré à l'individu ayant ce cancer, **caractérisé en ce que** la dite utilisation n'est pas une méthode de traitement du corps humain ou animal par chirurgie ou thérapie ou une méthode de diagnostic pratiquée sur le corps humain ou animal.

**25.** Kit comprenant une paire d'amorces conçues pour amplifier spécifiquement un acide nucléique ayant une séquence qui est spécifique au gène *plexine-B1*, **caractérisé en ce que** la séquence de la paire d'amorces est choisie entre la SEQ ID NO: 1 et la SEQ ID NO: 2.

**26.** Utilisation d'un kit comprenant une paire d'amorces conçues pour amplifier spécifiquement un acide nucléique ayant une séquence qui est spécifique au gène plexine-B1, pour détecter la présence d'un cancer du rein chez un individu, pour déterminer le stade de sévérité de ce cancer chez un individu, ou pour surveiller l'effet de la thérapie administrée à l'individu ayant ce cancer.

**27.** Utilisation d'un kit selon la revendication 26, **caractérisée en ce que** la séquence de la paire d'amorces est choisie entre la SEQ ID NO: 1 et la SEQ ID NO: 2.

FIGURE 1

FIGURE 2

# EP 1 642 133 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2004050914 A
- US 4683195 A
- US 4683202 A

### Non-patent literature cited in the description

- KOVACS G. et al. *J. Pathol.,* 1997, vol. 183, 131-133
- RITCHIE A.W.S. ; CHISHOLM G.D. *Semin. Oncol.,* 1983, vol. 10, 390-400
- PORENA M. et al. *J. Clin. Ultrasound,* 1992, vol. 20, 395-400
- KONNACK J.W. ; GROSSMAN H.B. *J. Urol.,* 1985, vol. 134, 1094-1096
- THOMPSON I.M. ; PEEK M. *J. Urol.,* 1988, vol. 140, 487-490
- KONNACK J.W. ; GROSSMAN H.B. *J. Urol.,* 1985, vol. 134, 1094-1096
- SMITH S.J. et al. *Radiology,* 1989, vol. 170, 699-703
- SEIZINGER B.R. et al. *Nature,* 1988, vol. 332, 268-269
- ISHIKAWA J. et al. *Int. J. Cancer,* 1990, vol. 45, 1018-1021
- LAGER D.J. et al. *Mod. Pathol.,* 1994, vol. 7, 544
- YAO M. et al. *Cancer Res.,* 1988, vol. 48, 6753-6757
- GUO X. et al. *Cancer Res.,* 2001, vol. 61, 2774-2781
- KUROIWA, K. et al. *J. Surg. Oncol.,* 2001, vol. 77, 123-131
- BOER J.M. et al. *Genome Res.,* 2001, vol. 11, 1861-1870
- ROBSON C.J. et al. *J. Urol,* 1969, vol. 101, 297-301
- HERR H.W. *Cancer,* 1994, vol. 73, 160-162
- RABINOVITCH R.A. et al. *J. Clin. Oncol.,* 1994, vol. 12, 206-212
- SANDOCK, D.S. et al. *J. Urol,* 1995, vol. 154, 28-31
- KJAER M. et al. *Int. J. Radiat. Oncol. Biol. Phys.,* 1987, vol. 13, 665-672
- DRIESSENS M.H. ; OLIVO C. ; NAGATA K. ; INA-GAKI, M. ; COLLARD J.G. *FEBS lett.,* 2002, vol. 529, 168-172
- CASTELLANI V. et al. *Curr. Opin. Neurobiol.,* 2002, vol. 12, 532
- KEELY et al. *Nature,* 1997, vol. 390, 632-637
- SANDER et al. *J. Cell Biol.,* 1998, vol. 143, 1385-1398
- VIKIS H.G. ; LI W. ; GUAN K.L. *Genes Dev.,* 2002, vol. 16, 836-845
- VIKIS H.G. ; LI W. ; GUAN K.L. *Proc. Natl. Acad. Sci. USA,* 2002, vol. 99, 12085-12090
- GIESEG M.A. ; CODY T ; MAN M.Z. ; MADORE S.J ; RUBIN M.A. ; KALFJIAN E.P. *BMC Bioinformatics,* 2002, vol. 3, 26
- CHOMCZYNSKI P. et al. *Anal. Biochem.,* 1987, vol. 162, 156
- CHOMCZYNSKI P. *Biotechniques,* 1993, vol. 15, 532
- KATZAV S ; MARTIN-ZANCA D ; BARBACID M. *EMBO J,* 1989, vol. 8, 2283-2290